# EUROPEAN PATENT APPLICATION

(11) **EP 1 053 750 A1**
(43) Date of publication of application: **22.11.2000**
(21) Application number: 99107993.0
(22) Date of filing: 22.04.1999
(51) Int. Cl.: A61K 38/06, A61K 38/07

(54) **Use of proteasome-inhibitor for the induction of programmed cell death (apoptosis)**

(71) Applicant: Max-Planck-Gesellschaft zur Förderung der Wissenschaften e.V., 80539 München (DE)
(72) Inventor: Drexler, Johannes, 61200 Wolfersheim (DE); Konerding, Moritz A., Prof.Dr., 55126 Mainz (DE); Risau, Werner, Dr., verstorben (DE)
(74) Representative: Weiss, Wolfgang, Dipl.-Chem. Dr.

(57) **Abstract**

The present invention relates to a method of selectively inducing a programmed cell death (apoptosis) in activated endothelial cells or leukemic cells having a high expression level of c-myc by the use of proteasome-inhibitor.

## Description

The present invention relates to a method of selectively inducing a programmed cell death (apoptosis) in activated endothelial cells or leukemic cells having a high expression level of c-myc.

Apoptosis is an essential component of diverse biological processes such as embryonic development or tissue homeostasis in multicellular organisms. Once programmed cell death is initiated, a well-defined proteolytic system, namely the caspase family of cysteinyl proteases, becomes activated inside the cell in a cascade like process. Members of the caspase family are responsible for the site-specific cleavage of various proteins representing key structural or regulatory elements of the cell. Caspase activation and caspase mediated proteolysis is a hallmark of apoptosis and contributes, together with the fragmentation of cellular DNA, to the irreversibility of the process of cell death (reviewed in Lin et al., Cell 89 (1997), 175 - 184; Thornberry and Lazebnik, Science 281 (1998), 1312 - 1316).

In principle all cells have the potential to undergo apoptosis, and in most cells apoptosis appears to be independent of the synthesis of new proteins. This indicates that all components of the death machinery including the caspase precursor molecules are constitutively expressed in these cells and that the apoptotic machinery is activated only upon the cell encountering an appropriate stimulus (Weil et al., J. Cell Biol. 133 (1996), 1033 - 1059).

The ubiquitin-proteasome system is interlinked with both apoptotic cell death and caspase activation (Delic et al., British J. Cancer 77 (1998), 1103 - 1107; Drexler, Proc. Natl. Acad. Sci. USA 94 (1997), 855 - 860; Fujita et al., Biochem. Biophys. Res. Comm. 224 (1996), 74 - 79; Grimm et al., EMBO J. 15 (1996), 3835 - 3844; Imajoh-Ohmi et al., Biochem. Biophys. Res. Comm. 17 (1995), 1070 - 1077, Lopes and Cooper, Mol. Biol. Cell 7 (1996), 30A; Sadoul et al., EMBO J. 15 (1996), 3845 - 3852; Shinohara et al., Biochem. J. 317 (1996), 385 - 388). The ubiquitin-proteasome system is responsible for various intracellular proteolytic processes which are critical not only for the rapid elimination of misfolded or damaged proteins, but also for the regulation of cell cycle progression and cell proliferation, and for the regulation of gene activation as well as for the generation of certain antigenic peptides (reviewed in Feneteany and Schreiber, J. Biol. Chem. 273 (1998), 8545 - 8848; Hershko, Curr. Opin. Cell Biol. 9 (1997), 788 - 799; Hochstrasser, Ann. Rev. Genet. 30 (1996), 405 - 439). Intracellular proteins are targeted for proteasomal degradation with high selectivity by their conjugation with multiple ubiquitin molecules and their subsequent binding and degradation by the 26S proteasome. The 26S proteasome, a giant multisubunit protease complex has been found to be associated with the nucleus, the endoplasmatic reticulum and the cytoplasm. The interplay between multiple enzymes that selectively recognize and ubiquitinate substrate proteins and the cleavage process, which is compartmentalized within the proteasomal cavity, ensures a highly ordered pathway to proteolysis, and at the same time prevents random degradation of other cytosolic proteins.

The interference with proteasomal function by specific inhibitors consitutes one of the signals which is capable of engaging the death machinery in different tumor cell types. Thus, apoptosis can be induced in tumor cell lines U937, MOLT-4, L5178Y, Rvc lymphoma and HL60 cells by different proteasomal inhibitors such as lactacystin, LLnL, LLnV or PSI (Drexler (1997), supra; Fujita et al. (1996), supra; Imajoh-Ohmi et al. (1995), supra; Shinohara et al. (1996) supra; Tanimoto et al., J. Biochem. 121 (1997), 542 - 549).

To date only immortalized cell lines or tumor cells have been used to study the induction of apoptosis by proteasomal inhibitors. In this study we have attempted to delineate the link between the proliferative status of endothelial cells and apoptosis induction by two proteasomal inhibitors in vitro and in vivo. Endothelial cells are central to the pathophysiology of various disease states which are characterized by extensive formation of new blood vessels (for review see Folkman, Nature Medicine 1 (1995), 27 - 31), such as tumor growth, diabetic retinopathies or rheumatoid arthritis. In these conditions, quiescent endothelial cells within established blood vessels become activated by the action of paracrine growth factors such as vascular endothelial growth factor (VEGF), reenter the cell cycle and resume proliferation (Kim et al., Nature 362 (1993), 841 - 844; Millauer et al., Cancer Res. 56 (1996), 1615 - 1620, Millauer et al., Nature 367 (1994), 576 - 579; Plate et al., Nature 359 (1992), 845 - 848). This situation is closely recapitulated by endothelial cells in vitro: They remain within the cell cycle when kept under subconfluent culture conditions but become largely quiescent when reaching confluency, thus providing an experimental system to address the question of differential sensitivities towards proteasomal inhibition. In this study the chorioallantoic membrane (CAM) of the developing chick embryo was selected as a complementary in vivo assay system. The CAM is a densely vascularized extra-embryonic membrane which has been used for many years to investigate the effect of a variety of compounds on the formation of new blood vessels (Folkman, Cancer Res. 34 (1974), 2109 - 2113) and for studies targeted at the mechanisms of tumor cell invasion (Kim et al., Cell 94 (1998), 353 - 362).

Compounds which preferentially kill proliferating endothelial cells but leave the quiescent endothelium unharmed would be extremely usefull in any therapeutic approach involving the vasculature. Here we present evidence to support the assertion that induction of endothelial apoptosis by proteasome inhibiting compounds represent a useful approach in the preparation of novel anti-angiogenic and anti-neoplastic strategies.

A subject matter of the present invention is the use of a proteasome-inhibitor for the preparation of an agent for inducing apoptosis in proliferating endothelial cells or epithelial cells, wherein said proteasome-inhibitor is preferably represented by the general formula (I):

Z - R₁ - R₂ - R₃ - R₄ - X

wherein R₁ is selected from leucine, valine and isoleucine, R₂ is selected from glutamine, glutamic acid and blocked derivates thereof, R₃ is selected from alanine and glycine and R₄ is selected from leucine, valine and isoleucine, Z is an amino-group or a protected amino-group and X is a carboxy-group, an aldehyde group, a sulfon group and a boronate group. The amino-acids R₁, R₂, R₃ and R₄ in the peptidic compound (I) are preferably L-amino-acids. R₁ is preferably isoleucine, R₂ is preferably a glutamic acid ester, particularly glutamic acid-t-butyl ester, R₃ is preferably alanine and R₄ is preferably leucine. The amino terminal group Z is preferably a protected amino group, e. g. carbobenzoxy, benzoylglycine, t-butoxycarbonyl or acetyl. More preferably, Z is a carbobenzoxy group. The carboxy terminal group X is preferably an aldehyde group, a boronate group e. g. as described in US-A-5,693,617 or a vinyl sulfon group. In a particularly preferred embodiment of the present invention the compound (I) iscarbobenzoxy-L-isoleucyl-*y*-t-butyl-L-glutamyl-L-alanyl-L-leucinal (PSI).

Surprisingly it was noticed that compounds of the general formula (I) can activate apoptosis by inhibiting the proteasomal activity selectively in proliferating endothelial or epithelial cells, particularly in proliferating endothelial cells. This is shown by reduction of the proteasomal activity, accumulation of the cdk inhibitor p27^{Kip 1}, characteristical morphologic changes of the cells, DNA laddering, fragmentation of the nucleus, detection of the activity of caspase-3 and caspase-1 and inhibition of cell death by a caspase-3 specific inhibitor as well as a proteolytical cleavage of poly(ADPribose)polymerase (PARP) and β-catenin. For contact inhibited quiescent endothelial or epithelial cells a significantly higher concentration of the compound (I) is necessary, preferably higher by the factor 10, more preferably by the factor 50 at least and most preferably by the factor 100 at least in order to obtain 50% cell death. This proves that the compounds (I) are preferentially toxic for proliferating endothelial or epithelial cells. The use of compounds (I) in vivo in a tissue of a high cell cleavage rate leads to a functional tissue avascularity and to a general induction of apoptosis. This is demonstrated in experiments with the chorioallantoic membrane of a developing chick embryo.

Compounds of the formula (I) thus have anti-mitogenic and anti-angiogenic characteristics which can either be directly used to produce therapeutics for the treatment of angiogenic diseases or for the further development of such therapeutics. Specific fields of application are measures to combat diseases such as tumors, diabetic retinopathy, rheumatoidic arthritis, psoriasis, AIDS etc. The compounds (I) further react on humane leukemia cells with the induction of apoptosis, namely especially leukemias which are characterized by a high expression level of c-myc.

The compounds (I) can be administered in any known way, for example by oral, parenteral, pulmonal or topic application. The compounds of the formula (I) are transferred to a pharmaceutically acceptable formulation by ususal pharmaceutical carriers, adjuvants ingredients or diluents. Depending on the application form, this formulation can be a solution, a tablet, a coated tablet, a cream, an ointment or an aerosol. Apart from the compound (I), depending on requirement, further active substances to combat the disease to be treated can be applied together with the compound (I) or in separate forms of administration. The height of the dosage and the duration of the administration depends on the severity of the disease.

Thus the present invention also refers to a method for inducing apoptosis in proliferating endothelial or epithelial cells comprising administering a subject in need thereof, preferably a human, a therapeutically effective amount of a proteasome-inhibitor which has a selectivity for proliferating endothelial or epithelial cells versus quiescent endothelial or epithelial cells. Preferably the proteasome-inhibitor is represented by the general formula (I) as defined above.

Although the induction of the apoptosis by proteasome-inhibitors in leukemia cells is already described in Drexler (Proc. Natl. Acad. Sci. USA 94 (1997), 855 - 860), there is no hint on the application for leukemias which are characterized by a high expression level of c-myc. Examples for such leukemias are Burkitt lymphoma, plasmacytoma and multiple myeloma.

Thus, a further subject-matter of the present invitation is the use of a proteasome-inhibitor for the manufacture of an agent for inducing apoptosis in leukemic cells having a high expression level of c-myc, wherein said proteasome-inhibitor is represented by the general formula (I) as defined in claim 1.

Further, the invention is explained by the following examples and figures.

### Figure 1

Induction of apoptosis in subconfluent primary bovine aortic endothelial cells (BAE) and human umbilical vein endothelial cells (HUE) cells by PSI. BAE (a - d) and HUE (e - h) cells were treated for 18 - 24 hours with either 0.1 % DMSO (a, e) or 50 µM PSI (c, g) and then stained with Hoechst 33342 to reveal nuclear morphology. Phase contrast pictures are shown on the left, and identical areas which display the nuclear morphology after Hoechst staining are shown on the right. Arrowheads point at nuclei with apoptotic morphology. Scale bars are 50 µm.

### Figure 2

a. Analysis of PSI-induced DNA fragmentation by agarose gel electrophoresis. HUE cells treated with 10 µM PSI for 18 hours induced DNA ladder formation (lane 1), which could be blocked by an excess of caspase-3 inhibitor Ac-DEVD-CHO (250 µM, lane 2), but not by the caspase-1 inhibitor Ac-YVAD-CHO (250 µM, lane 3). Neither caspase inhibitor (Ac-YVAD-CHO 250 µM, lane 4; Ac-DEVD-CHO 250 µM, lane 5) nor DMSO (lane 6) induced the characteristic pattern of DNA fragmentation. M1 and M2: DNA size markers (kb).
b. Immunoblot analysis of HUE cell lysates after incubation with PSI. Lysates of HUE cells (30 µg) which had been treated with 50 µM PSI for the times indicated, were separated on 10 or 12% SDS-polyacryamide gels, transferred to nitrocellulose and probed with antibodies recognizing p27^{Kip 1}, PARP, β-catenin, bcl-2, bcl-xL and bad as described in Materials and Method. Equal loading was controlled by using an antibody directed against GAPDH enzyme. Accumulation of p27^{Kip 1} indicates proteasome inhibition, while processing of PARP and β-catenin demonstrate the presence of caspase-3-like activity.

### Figure 3

Proteasomal activity is reduced in PSI and LLnV treated cell lysates. Lysates prepared from BAE cells incubated for 15 hours with 50 µM of the indicated inhibitors were assayed for their ability to suppress proteasomal function by using Suc-LLVY-AMC as substrate for the chymotryptic activity (a) and Z-LLE-AMC as substrate for the post acidic residue cleaving activity (b). Activities are expressed as the relative fluorescence measured after 45 min of incubation with the substrates and reflect the mean of two independent experiments each performed in duplicate.

### Figure 4

Detection of a caspase-3-like activity in lysates of PSI-treated HUE cells. Subconfluent endothelial cells were incubated with the inhibitors for the indicated times, lysed and caspase activities determined using Ac-DEVD-AMC for caspase-3-like enzymes and Ac-YVAD-AMC for caspase-1-like activities as described in the Material and Methods section (PSI, filled circles; LLnM, open square; E64, open triangles; Leupeptin, filled triangles; Pepstatin, filled squares). The specific caspase activities are shown as pmol of substrate hydrolyzed/min per µg of protein.

### Figure 5

Endothelial cell viability is reduced after incubation with proteasome inhibitors. The viability of cells was measured in subconfluent cells (left column) or cells grown to confluency (right column) after incubation with various inhibitors: PSI (filled circles); LLnV (open squares); E64 (open triangles); Leupeptin (closed triangles); Pepstatin (filled squares) by an MIT assay. The indicated values are expressed as the percentage of absorbance at 570 nm relative to the absorbance obtained by DMSO treated (control) cells.

### Figure 6

Confluent endothelial cells display a reduction in S-phase cells. Subconfluent BAE (a) or HUE cells (b) as well as confluent cultures of BAE (c) and HUE cells (d) were analyzed by PI staining and FACS analysis to determine their cell cycle distribution. Inserts give the corresponding percentage of cells in the G0/G1 and S-phase of the cell cycle.

### Figure 7

BrdU labeling of cells within the chick embryo chorioallantoic membrane. (a) Continuous BrdU labeling of CAM tissue for 24 h. Numerous cells that have incorporated BrdU are detected in particular in the subepithelial layer of cells, which harbours the dense capillary plexus of the CAM. Labeled endothelial nuclei are marked by arrowheads. (b) Control section; anti-BrdU antibody omitted in staining procedure. Scale bars are 50 µm. (c and d) PSI-induced apoptosis in the CAM tissue. Arrowbars point at numerous cell nuclei which display an apoptotic phenotype as judged by double-staining with the TUNEL technique (c) and with Hoechst 33342 (d). The lumen of a large vessel containing blood cells with a strong Hoechst 33342 fluorescence is marked by a circle. The nuclei of the blood cells are not apoptotic and give only background staining with the TUNEL technique. Scale bars are 100 µm.

### Figure 8

PSI triggers vascular regression in the CAM vasculature. (a) Luconyl Blue injection into the vascular system of a chick embryo to examine vascular perfusion efficacy after PSI-treatment of CAM. The area which displays lack of perfusion is indicated by arrowheads. (b) CAM with DMSO containing control disc. The circumference of the disc is indicated by an interrupted line. (c) Corrosion cast of PSI-treated CAM and of control CAM, treated with DMSO only (d). (e) Semi-thin section through a CAM which had received a PSI containing polymer disc. Arrowheads indicate endothelial cells with cuboidal morphology. Numerous pycnotic nuclei are detected. (f) Semi-thin section through control CAM, receiving DMSO containing disc. Scale bars are 1 mm in (a) and (b), 100 µm in (c) to (f) and 10 µm in (g) and (h).

### Examples

### 1. Materials and Methods

### 1.1 Tissue Culture

Primary bovine aortic endothelial cells (BAE) were isolated from bovine pulmonary aorta according to the method of Schwartz (In Vitro 14 (1978), 966 - 980) and maintained in MDCB131 medium supplemented with 8% fetal calf serum (FCS), penicillin (100 lU/ml) and streptomycin (100 µg/ml) at 5% CO₂. Human umbilical vein endothelial cells (HUE), an immortalized human umbilical vein endothelial cell line (ATCC CRL-1730) were cultured under the same conditions. Madin Darby canine kidney epithelial cells (MDCK ; ATCC CLL-34) were grown in Dulbecco's Modified Eagle's Medium (DMEM) containing 10% FCS, penicillin and streptomycin.

### 1.2 DNA fragmentation assay

HUE cells which had been plated at 20000 cells/cm² 24 hours before the assay, were treated with 250 µM of the caspase inhibitors Ac-YVAD-CHO or Ac-DEVO-CHO (Peptide Institute, Osaka, Japan) or the equivalent volume of dimethylsulfoxide (DMSO) for 1 h prior to additon of the proteasomal inhibitors. Incubation of the cells was continued for a further 18 h and the extent of DNA fragmentation was analyzed as previously described with some minor modifications (Hockenberry et al., Nature 348 (1990), 334 - 336). Briefly, both the floating cells and the adherent cells which were detached by trypsinization, were lysed in a solution containing 0.5% Triton X100, 10 mM EDTA and 10 mM Tris-HCL pH 7.5 for 30 min on ice. To separate the intact chromatin from the oligonucleosomal fragments, the lysate was centrifuged at 13000 g for 10 min at 4°C and the resulting supernatant was sequentially extracted with phenol followed by phenol/chloroform/iso-amylalcohol (25:24:1; v/v/v). Low molecular weight DNA was precipitated by the addition of 0.5 vol 7 M ammonium acetate and 2.5 vol ice-cold ethanol. The precipitate was washed in 75% ethanol, dissolved in TE-buffer, containing RNase A (20 µg/ml; Boehringer Mannheim), incubated for 30 min at 37°C and subjected to electrophoresis on 1.5% agarose gels.

### 1.3 Determination of proteasomal activity

BAE cells (1 x 10⁶) were plated onto 10 cm plastic dishes and treated for 16 h with either Leupeptin, E64, LLnM, LLnV or PSI (50 µM final concentration) which were diluted form 1000 x DMSO stock solutions. DMSO at a final concentration of 0.1% served as a vehicle-only control. Floating and adherent cells were harvested, lysed by sonication in ice-cold solution of 20 mM Tris-HCI pH 7.5, containing 20% glycerol, centrifuged in a benchtop centrifuge (10 min, 2000 g). The protein concentration of the resulting supernatant was determined by the bicinchoninic acid (BCA) assay (Smith et al., Anal. Biochem. 150 (1985), 76 - 85). A total of 15 µg of protein in a volume of 200 µl lysis buffer supplemented with 5 mM CaCl₂ was assayed for chymotryptic and peptidyl-glutamyl-hydrolyzing activity in the presence of 10 µM Suc-Leu-Leu-Val-Tyr-AMC (Bachem, Heidelberg, Germany) and 10 µM Z-Leu-Leu-Glu-AMC (Peptide Institute, Osaka, Japan) respectively.

### 1.4 Determination of caspase activities

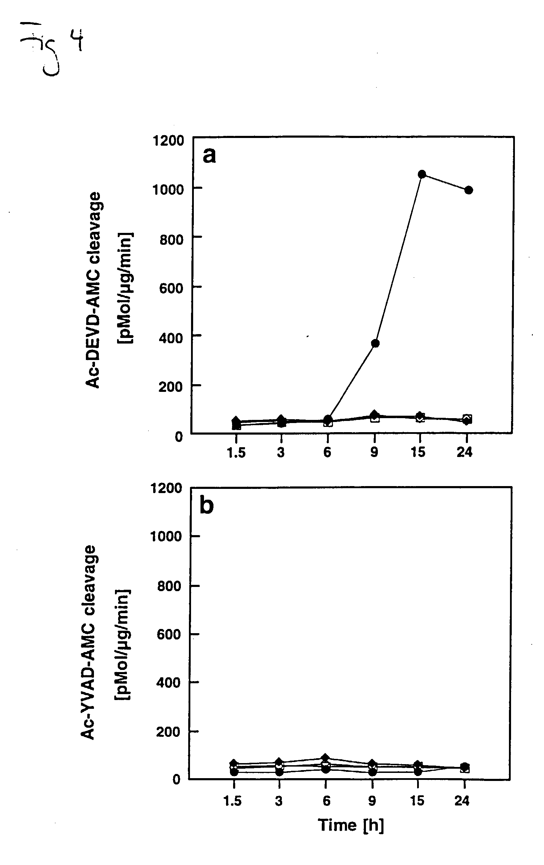

HUE cells (8 x 10⁵) were plated onto 10 cm plastic dishes, allowed to attach overnight, and inhibitors were added to a final concentration of 50 µM for the times indicated in Fig. 4. DMSO, which served as the vehicle, was added to a final concentration of 0.1%. Floating and adherent cells were then harvested, lysed in Buffer I (1% Triton X100, 10 mM KCl, 20 mM HEPES pH 7.4, 1.5 mM MgCl₂, 0.2 mM EGTA, 1 mM PMSF), snap frozen in liquid nitrogen and stored at -80°C until required. Lysates were centrifuged at 13000 g for 5 min at 4°C, the supernatants collected and protein concentrations determined using the BCA assay. A 40 µl aliquot of each sample was assayed in the presence of 20 µM Ac-DEVD-AMC or Ac-YVAD-AMC substrates (Bachem, Heidelberg, Germany) in Buffer II (50 mM PIPES-KOH pH 7.0, 0.1 mM EDTA, 1 mM DTT, 10% glycerol; total volume 250 µl) for 40 min at 37°C and the fluorescence intensity was measured with an excitation wavelength of 350 nm and emission wavelength of 430 nm. In addition, all samples were assayed in the presence of 10 µM Ac-YVAD-CHO and 1 µM Ac-DEVD-CHO respectively (Peptide Institute, Osaka, Japan) to exclude the possibility of non-specific substrate cleavage. Specific activity was expressed as pmol AMC liberated per min and per µg protein in comparison to a standard curve calculated from the amount of free AMC (SIGMA, Deisenhofen, Germany).

### 1.5 Western Blotting

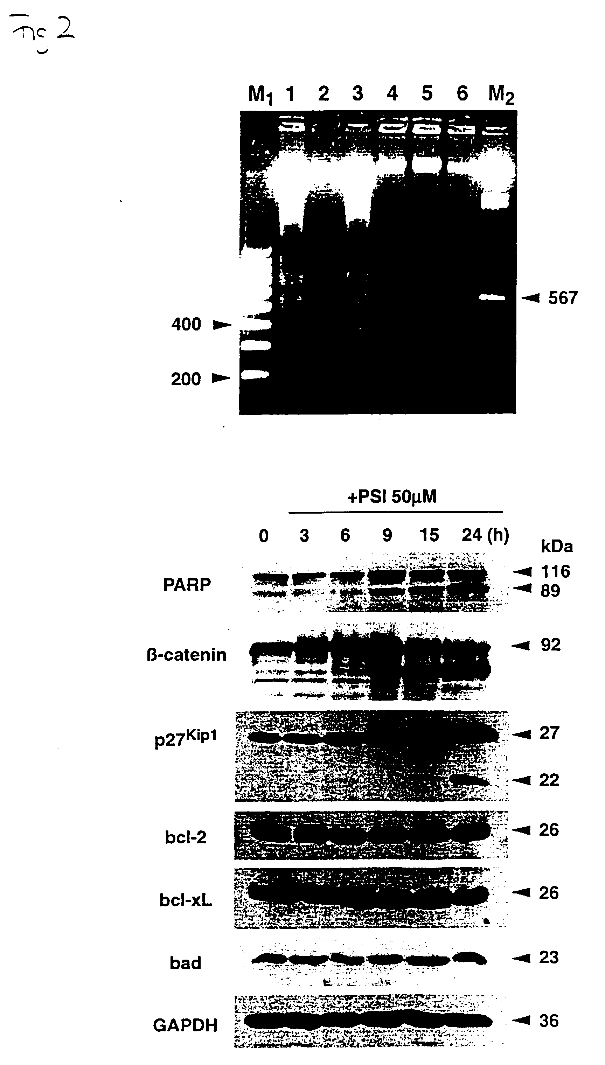

HUE cell lysates were prepared from cells treated with 50 µM PSI for the times indicated in Fig. 2B by lysing the cells in 1% SDS, 10 mM Tris-HCl pH 7.5 and denaturation at 95°C for 10 min. Equal amounts of protein were separated by 12% SDS-PAGE, transferred to nitrocellulose filters and blocked over night with TBS (50 mM Tris pH 7.5, 200 mM NaCI, 0.05% Tween 20), containing 5% non-fat dry milk-powder. Primary antibodies used in this study were obtained from DAKO (Bcl-2), Santa Cruz, Heidelberg, Germany (Bcl-xL, Bax), Transduction Laboratories, Dianova, Hamburg, Germany (p27^{Kip 1}, bad, β-catenin), Calbiochem, Bad Homburg, Germany (PARP) and Biogenesis, United Kingdom (glycerylaldehyde phosphate dehydrogenase; GAPDH), and incubated with the blots 1 h. After several washes with TBST, blots were incubated for 1 h with peroxidase-conjugated secondary antibodies (Dianova, Hamburg, Germany), washed again and developed with the SuperSignal chemoluminescence substrate (Pierce, KMF, St. Augustin, Germany).

### 1.6 Viability assay

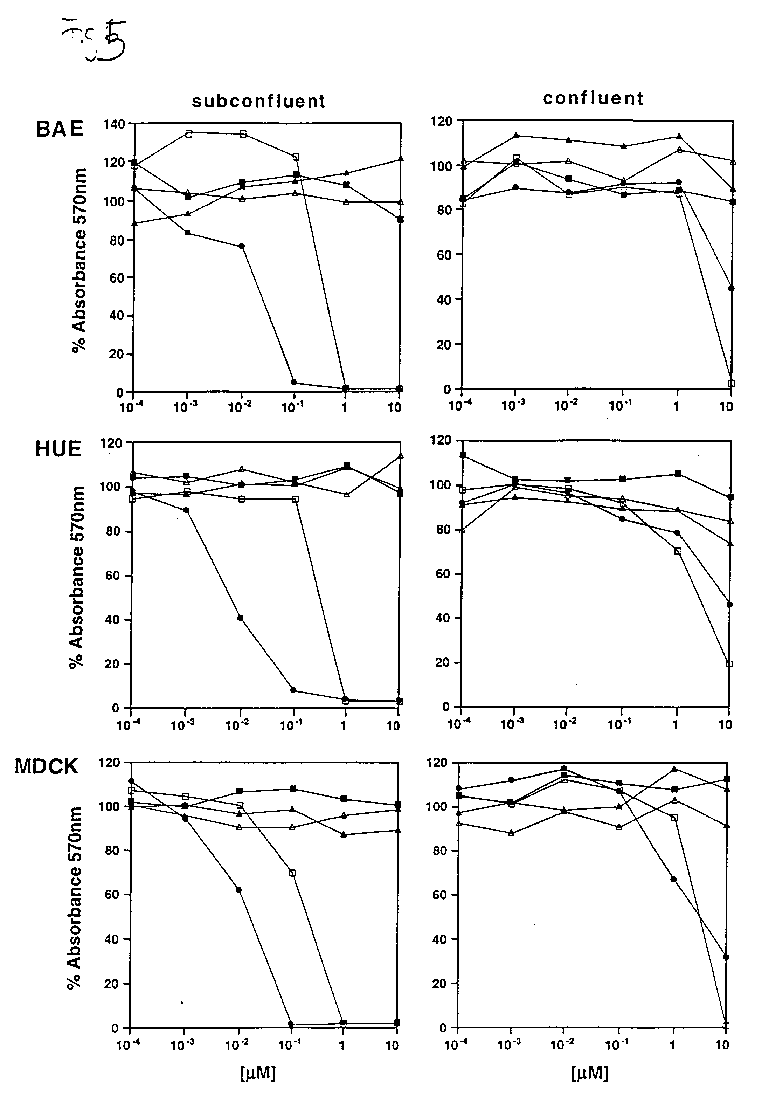

To determine the differential effect of proteasomal inhibitors on the viability of subconfluent cells, MIT assays were performed as previously described (Mosmann, J. Immunol. Meth. 65 (1983), 55 - 63). Briefly, the cells were plated either at 3000 cells/well (low density) or 30000 cells/well (high density) onto 96 well plates and grown over night. Protease inhibitors were then added in triplicate from 100-fold concentrated stock solutions prepared in DMSO at final concentrations between 100 pM and 10 µM (Fig. 5) to the low density cultures, and further incubated for additional 48 h prior to the addition of the MIT solution. The formazan cystals generated were subsequently dissolved in DMSO and absorption measured at 570 - 650 nm. High density cultures were grown to confluence for at least 4 days prior to the 48 h-incubation period with the protease inhibitors.

### 1.7 FACS analysis

Analysis of cell cycle distribution was performed as described (Nicoletti et al., J. Immunol. Meth. 139 (1991), 271 - 279). Briefly, both floating and adherent inhibitor-treated endothelial cells were harvested, resuspended in 1 ml of hypotonic fluorochrome solution (50 mg/ml propidiumiodide, 0.1 % sodium citrate, 0.1 % Triton X100) and placed at 4°C in the dark over night before flow-cytometric analysis using a FACScan flow cytometer and CellQuest and ModFit LT software (Becton Dickinson, Heidelberg, Germany).

### 1.8 Chorioallantoic membrane assays

All experiments with chick embryos were performed in ovo. After aspiration of 2 ml of eggwhite from the pointed side of the egg, a window was cut into the eggshell on embryonic day 3 or 4 (E3, E4). The eggs were resealed with tape and further incubated until E9. To obtain an indication of the number of cycling cells in the CAM tissue during the incubation period, a total of 4E9 chick embryos were injected with 25 mg/kg BrdU (Sigma) into an appropriate vein of the chorioallantoic membrane, returned to the incubator and cultivated for additional 24 h. Approximately 1 cm²-pieces of the CAM were then cut out, washed in PBS and fixed in Carnoy's solution. After dehydration and embedding in paraffin, 8 µm sections were cut and stained with an anti-BrdU mouse monoclonal antibody (Boehringer Mannheim, Germany) according to the manufacturer's instructions. Counterstaining of the sections was performed with Gill's hematoxylin (Serva, Heidelberg, Germany). Sections were mounted using Aquamount (Merck, Darmstadt, Germany) and photos were taken with a Zeiss Axiophot microscope. For the investigation of inhibitor efficacy on the CAM tissue, 1 part of a 5 mM PSI solution in DMSO was carefully added to a solution made by mixing 3.5 parts of a sterile 1 % methylcellulose solution and 2.5 parts sterile water. A series of 7 µl aliquots of this mixture, each containing a total amount of 3.1 µg of PSI were pipetted onto an bacteriological grade petri dish, air dried and placed onto E10 CAMs for 2 days. Control pellets were prepared with an equal amount of Leupeptin or with DMSO as vehicle, respectively. To aid in the visualization of the vascular system, embryos and CAMs were then injected using pulled glass capillaries with 10% Luconyl Blue (BASF, Ludwigshafen, Germany) in PBS, and photographs were taken using a stereomicroscope with camera adapter.

### 1.9 Microvascular corrosion casting and electron microscopy

The microvascular architecture of the CAM which underlied PSI- and control pellets was highlighted by means of microvascular corrosion casting. In eight eggs the windows of the egg shells were enlarged under an operation microscope. After a peripheral incision in the CAM was made, the main artery was incised with a pair of ocular scissors, and a glass capillary (World Precision Instruments, Inc. Germany) pulled to the adequate diameter, was inserted and sealed with a drop of cyanoacrylate (Loctite®). Efflux was enabled by making another incision into a main draining vessel. The vascular system was flushed with up to 5 ml of prewarmed (37 -39°C) physiological saline (0.9% NaCI) under mechanically controlled pressure. Fixation was performed with up to 5 ml of 2.5% phosphate-buffered glutaraldehyde (pH 7.40, 300 mosmol) over a period of 2 - 5 minutes. A methacrylate based acrylic resin solution was prepared by mixing 4 ml of Mercox CL-2B, 0.2 g of catalyst MA (Vilene Med. Co., Tokyo, Japan) and 1 ml of methylmethacrylate monomers (Merck, Darmstadt, Germany) and used as casting medium. After complete polymerization of the casting solution, the membranes were dissected and macerated in a solution of 5% potassium hydroxide (Fluka, Neu-Ulm, Germany) at 40°C for 1 - 2 days. The resulting corrosion casts of all specimens were freeze dried, mounted and sputter coated. Examination of the microvascular corrosion casts were carried out with a Stereoscan Mk-250 scanning electron microscope (Cambridge, England). Alternatively, in situ fixed CAM segments were cut out, processed for semi-thin sectioning and stained with methylene blue and Azur II. Selected specimens were trimmed for ultrathin sectioning, and counterstained with uranyl acetate and lead citrate.

### 2. Results

### 2.1 Proteasome inhibitors induce apoptotic cell death in endothelial cells

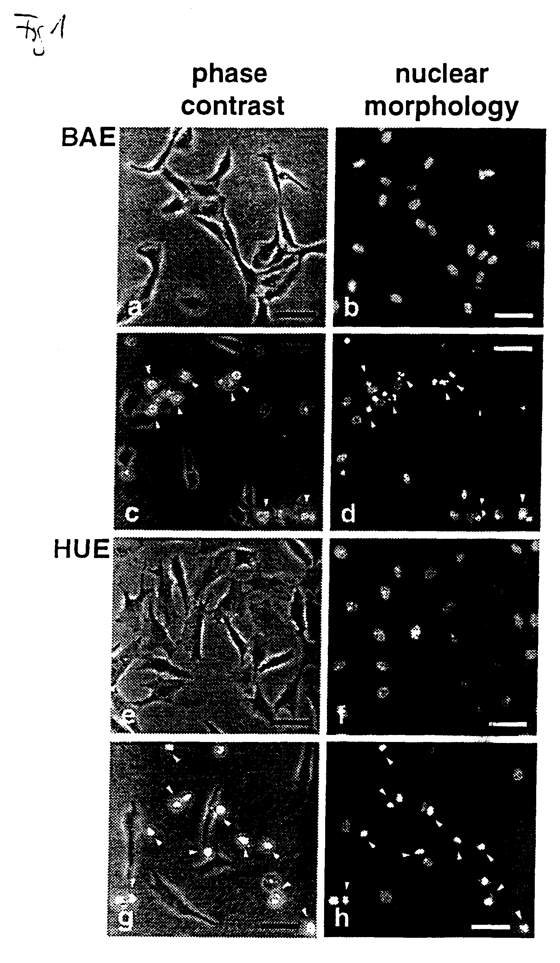

The incubation of subconfluent primary BAE cells with PSI resulted in several characteristic morphological changes associated with programmed cell death, such as cytoplasmic shrinkage and bleb formation as well as extensive nuclear condensation and fragmentation. An example of the morphological changes in BAE and HUE cells observed after treatment with 50 µM PSI for 15 - 24 h is shown in Fig. 1. Similar alterations of the cellular and nuclear morphology could be observed with 10 µM PSI after 48 hours of incubation. PSI-induced apoptosis was also linked to cleavage of chromatin into oligonucleosomal fragments (Fig. 2a, lane 1) as well as to the processing of poly(ADPribose)polymerase (PARP) and to that of beta-catenin which are substrates for caspase-3 and caspase-6 respectively (Fig. 2b). Two other inhibitors of proteasomal function, LLnV (MG115) as well as LLL (MG132), had the same effect.

In contrast, the addition to cultured BAE cells of LLnM, which is a potent calpain inhibitor (Kᵢ = 9 nM) and cathepsin B inhibitor (Kᵢ = 12 nM), but only a weak inhibitor of proteasomal function (Rock et al., Cell 78 (1994), 761 - 771), did not induce any changes in cellular morphology or viability.

Cell death induction experiments with proteasomal inhibitors were carried out both in BAECs and in an immortalized human umbilical vein endothelial cell line (HUE) in order to confirm that the observed effects (morphological changes, nuclear condensation and fragmentation, DNA fragmentation, reduction of S-phase cells upon contact inhibition) were not specific for one cell type only. The HUE cells were also included for the western blotting experiments as several antibodies available for this study did not show crossreactivity with the corresponding bovine antigens.

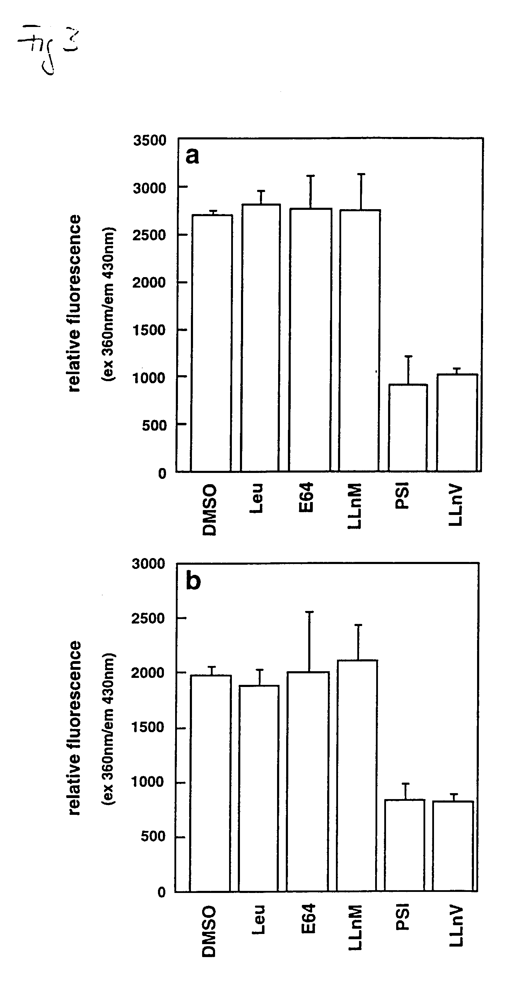

In total cell lysates, which were prepared after a 15 h incubation of BAECs with inhibitors, cleavage of Suc-Leu-Leu-Val-Tyr-AMC, a substrate for the chymotrypic activity of the proteasome, was clearly reduced with PSI and LLnV, but not with Leupeptin, E64 or the calpain inhibitor LLnM (Fig. 3a). Also, the total cellular peptidyl-glutamyl hydrolyzing activity of the proteasome, which was determined by using Z-Leu-Leu-Glu-AMC as a substrate, was similarly reduced only by the addition of PSI and LLnV, but not by any of the other inhibitors (Fig. 3b). Further evidence that the proteasomal function is blocked by the action of PSI is derived from the observation that the relative protein levels of the cystein-dependent kinase inhibitor p27_{Kip 1} start to accumulate between 6 and 9 h after the addition of the proteasome inhibitors (Fig. 2b).

The anti-p27^{Kip1} antibody we used detected a band of approximately 22 kDa in the HUE cell lysates in the samples collected at 15 and 24 h after addition of the inhibitor. Cleavage of the ckis p21 and p27 has been linked to the induction of apoptosis in HUVECs subjected to growth factor deprivation (Levkau et al., Molecular Cell. 1 (1998), 553 - 563), further supporting the proposal that endothelial cell death follows an apoptotic pathway. The presence of DPSD motif at amino acids 136 to 139 of the p27^{Kip1} sequence argues in favor of such a cleavage by a caspase-3-like activity. In contrast to p27^{Kip1}, no change in the relative amounts of either GAPDH, the antiapoptotic proteins bc12 and bcl-xl or the pro-apoptotic protein bad are observed in lysates of PSI-treated cells (Fig. 2b).

### 2.2 PSI-induced apoptosis in endothelial cells can be blocked by Ac-DEVD-H but not by Ac-YVAD-H

Here we show that a caspase-3 like enzyme becomes activated during PSI-mediated endothelial cell apoptosis. The cleavage of the fluorigenic caspase-3 substrate Ac-DEVD-AMC was detected in HUE cell lysates between 6 and 9 h after addition of the proteasome inhibitor PSI and reached a maximum at 15 h (Fig. 4a) which is consistent with the observed kinetics for PARP and beta-catenin cleavage in HUE cells (Fig. 2b). In contrast, caspase-1 like activity could not be detected throughout the course of these experiments (Fig. 4b). The rise in caspase-3 like activity (Fig. 4a) was not due to non-specific substrate hydrolysis by the total cell lysate as inclusion of the caspase-3 inhibitor Ac-DEVD-CHO in the assay mixture completely abrogated the measured caspase-3 like activity to background levels.

From these results we propose that the induction of apoptosis in endothelial cells by proteasome inhibitors occurs independently of caspase-1 like family members, but is mediated by a caspase-3 like activity. The specific involvement of a caspase-3 like enzyme in the proteasome inhibitor induced endothelial cell death was in addition supported by the observation, that only the caspase-3 inhibitor Ac-DEVD-CHO blocked the morphological changes as well as the DNA fragmentation which occured as a result of the PSI treatment (Fig. 2a, lane 2). The caspase-1 inhibitor Ac-YVAD-CHO, in contrast, was unable to inhibit the action of PSI (Fig. 2a, lane 3). None of the two caspase inhibitors alone, nor the solvent DMSO displayed any effect on the pattern of DNA fragmentation in endothelial cells (Fig. 2b, lanes 4 - 6).

### 2.3 Proliferating and confluent endothelial cells display differential sensitivity toward inhibition of proteasome function

In order to determine whether endothelial cells repond differently toward proteasome inhibition when either actively proliferating or in a contact inhibited, quiescent state, cellular viability was assessed by measuring the capacity of the cells to reduce the tetrazolium salt MIT (Fig. 5). A dramatic loss of viability was observed in BAE cells at low cell density, which had been incubated for two days with PSI (EC₅₀ = 24 nM), Cellular viability was also reduced, albeit to a lesser degree with LLnV (EC₅₀ = 402 nM). Similar results were obtained at low density with HUE cells (EC_{50, PSI} = 7 nM; EC_{50,} _{LLnV} = 310 nM) and also with MDCK epithelial cells (EC_{50, PSI} = 16 nM; EC_{50,} _{LLnV} = 196 nM) indicating that apoptosis is effectively induced in proliferating cells at rather low concentrations of PSI and LLnV. Furthermore, the observed proapoptotic effect is not specific for endothelial cells but also affects other epitheloid cell types, such as MDCK cells. In contrast, BAE cells which had been seeded at high density and were grown to confluency for 4 days displayed a greatly reduced sensitivity towards treatment with PSI or LLnV (EC_{50, PSI} = 8.15 µM; EC_{50, LLnV} = 2.72 µM) : a 340 fold higher concentration of PSI and a 6.8 fold higher concentration of LLnV were necessary to achieve a 50% reduction of viability. Similar results were obtained with HUE cells grown to confluency (1119 fold for PSI; 8.1 fold for LLnV) and also with confluent MDCK cells (194 fold for PSI, 15.1 fold for LLnV), when assayed under the same conditions. Table I summarizes the results of the viability assays.

**Table I**

| cell type | EC_{50,PSI} [nM]* | x difference | EC_{50,LLnV} [nM]* | x difference |
|---|---|---|---|---|
| BAE, sub; | 24 | | 402 | |
| BAE, con | 8150 | 340 | 2720 | 6.8 |
| HUE, sub; | 7 | | 310 | |
| HUE, con | 7830 | 1119 | 2500 | 8.1 |
| MDCK, sub; | 16 | | 196 | |
| MDCK, con | 3100 | 194 | 2950 | 15.1 |

| | | | | |
|---|---|---|---|---|
| *The EC₅₀-value was defined as the inhibitor concentration at which cellular viability was reduced to 50% of the DMSO control value. Coordinates corresponding to the EC₅₀-values for PSI and LLnV were derivated from the graphs shown in Fig. 5 using the identify tool of Kaleidagraph 3.0.2 software. | | | | |

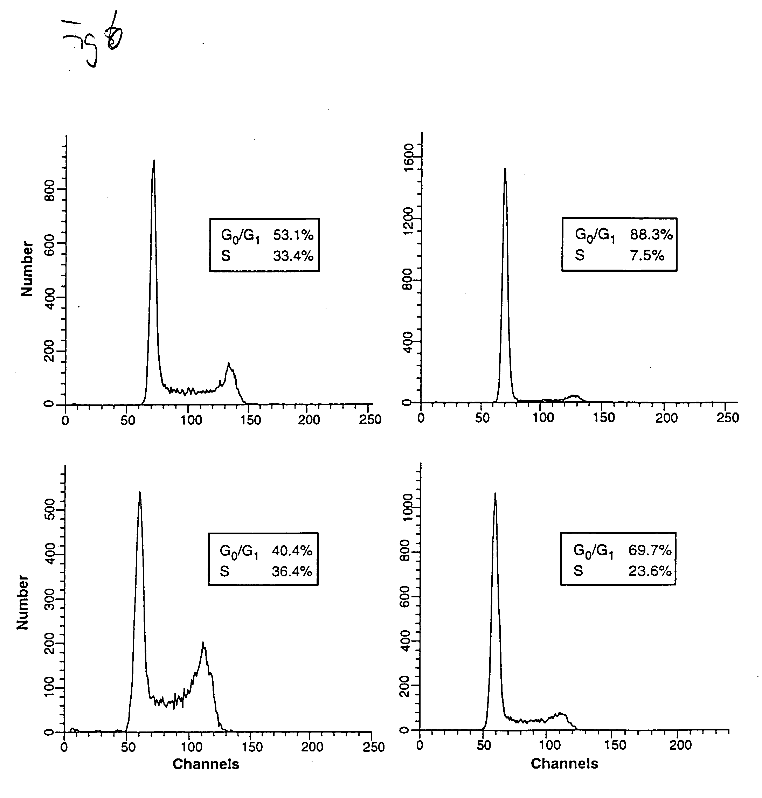

The effect of contact inhibition of endothelial cells on cell cycle distribution was confirmed by comparing confluent endothelial cell cultures and e dothelial cells plated at low density by using propidiumiodide staining and FACS analysis. The percentage of BAE cells which are within the S-phase compartment of the cell cycle is reduced by nearly 80% in confluent cultures compared to BAE cells growing at low density (Fig. 6) while the reduction of HUE cells in S-phase upon reaching confluency was less pronounced.

We concluded from these observations that inhibition of proteasomal activity has a pro-apoptotic effect primarily in endothelial cells that are in a proliferative state and that non-proliferating endothelial cells are much more resistant to the inhibitor effect. Significantly higher doses of proteasome inhibitors are necessary to achieve a similar degree of apoptosis in contact inhibited endothelial cells.

### 2.4 Proteasome inhibitors induce vascular regression and cell death in vivo

In order to examine whether the inhibition of proteasomal activity also exerts a pro-apoptotic effect on a proliferating tissue in vivo, a series of CAM assays were carried out. As the results of our in vitro experiments indicate that inhibition of proteasome function will initially affect proliferating endothelial cells, we first wanted to determine which, and how many cells of the CAM would be entering the S-phase of the cell cycle during an extended incubation period, similar to the one used for the inhibitor-loaded methylcellulose discs (see below).

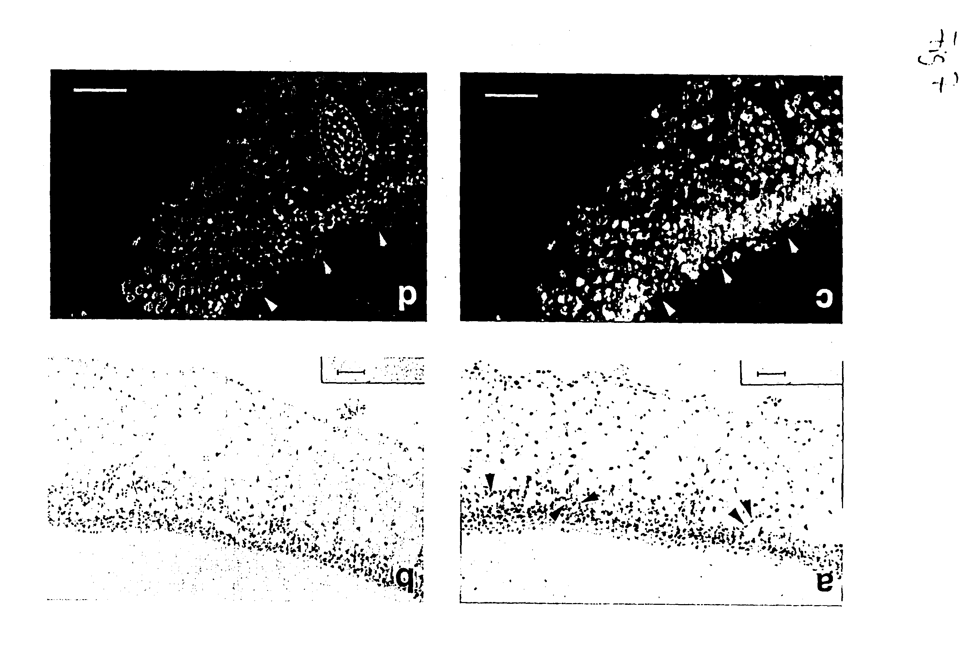

Chick embryos were therefore labeled continuously for 24 h with BrdU and following processing and cutting, the paraffin sections of the CAMs were then analyzed for the locations of BrdU incorporation. Numerous endothelial cell nuclei of vessels within all CAM layers were labeled after this period, apart from cells in the chorionic and allantoic epithelium of the CAM and apart from mesenchymal cells (Fig. 7a). Even individual nuclei of endothelial cells within the wall of large caliber vessels were labeled indicating that although these vessels with a considerably larger diameter have a more mature vessel wall composition than capillaries and show an increased state of quiescence, endothelial proliferation has not come to a complete halt yet.

It was obvious from these results that any compound which was applied to the CAM for a period of more than 24 hours would encounter numerous cycling endothelial as well as epithelial and mesenchymal cells.

Next, PSI, Leupeptin or DMSO containing methylcellulose discs were placed onto the CAM of E9 embryos for 48 h and then analyzed by a variety of techniques. Paraffin sections of PSI- and DMSO-treated CAMs were stained with the TUNEL technique and the nuclear dye Hoechst 33342. Intense labeling of cell nuclei with the TUNEL technique was observed in cells which had been in close contact with the PSI-containing methylcellulose disc (Fig. 7c), with the proportion of TUNEL labeled cells diminishing as the cell layers more distant to the disc are examined. Cells which gave a positive signal with the TUNEL technique were also found to contain condensed or fragmented nuclei when sections were doublestained with the nuclear dye Hoechst 33342 (Fig. 7d), indicating that cell death in fact occurred by an apoptotic process. In contrast, in sections of DMSO treated control CAMs apoptotic cells could be detected only with low frequency.

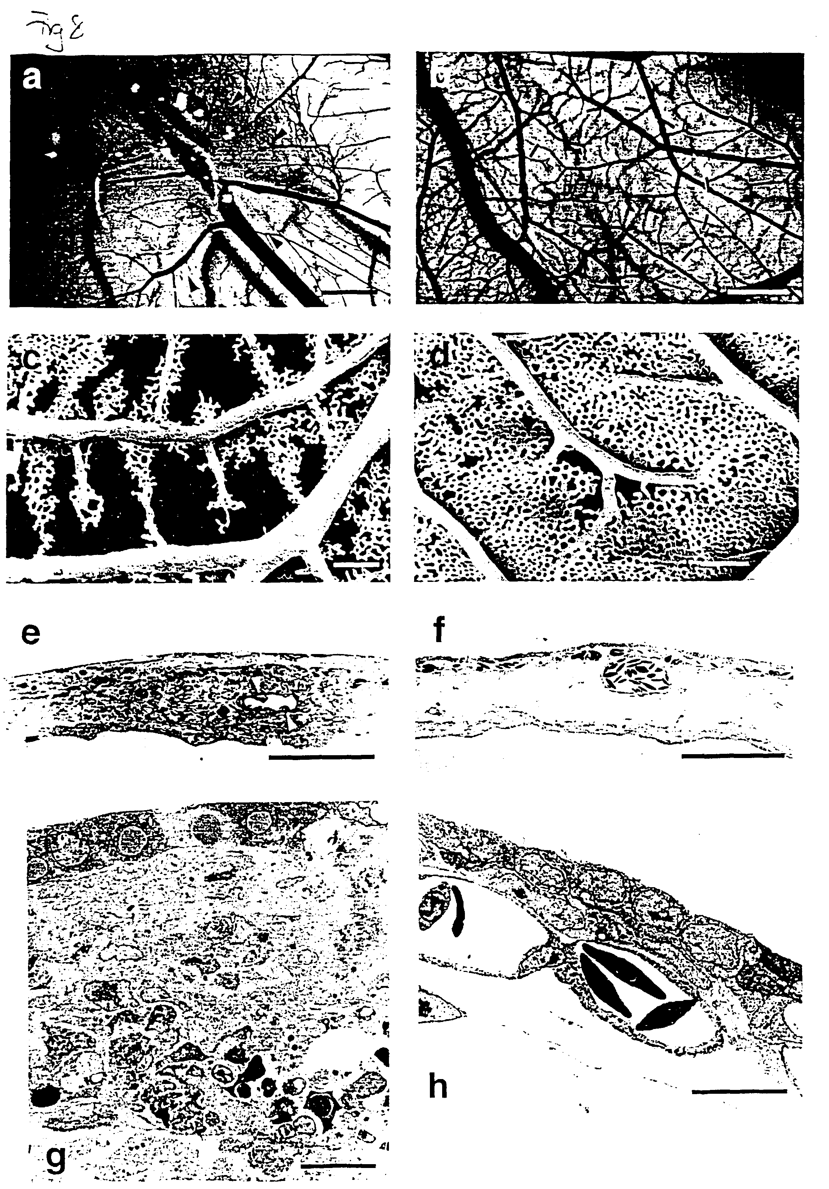

Morphological examination as well as injection of a dye into the embryonic vascular system, revealed that entry of the dye into the vessels of the area beneath the disc was prevented and that smaller vessels in particular were no longer efficiently perfused (Fig. 8a). In several CAMs, small capillaries with a regular vessel wall architecture could no longer be detected by morphological analysis. Larger vessels within these CAMs were not affected to the same extent as capillaries as access of the dye to the lumen of these vessels was apparently not occluded. In some instances the gross morphological appearance of large caliber vessels appeared distorted though.

DMSO which was used as solvent control as well as the same amount of Leupeptin, a tripeptide protease inhibitor containing a carboxyterminal aldelyde group similar to PSI or LLnV, did not elicit any significant changes in the vascular patterning or perfusion efficacy in the CAM (Fig. 8b). Corrosion casts show that in contrast to DMSO containing control discs (Fig. 8d), that in the area of the CAM directly located under the PSI-containing discs the densities of the capillary plexus as well as those of the first order vessels are rarified (Fig. 8c). Semi-thin sections of CAMs exposed to PSI (Fig. 8e), but not in those exposed to DMSO (Fig. 8f) revealed numerous pycnotic nuclei or nuclear fragments and further support the notion that PSI induced massive apoptosis in the CAM tissue. In individual vessels endothelial cells with a cuboidal cell shape are detected (Fig. 8e). These endothelial cells have lost the intercellular contact to neighbouring endothelial cells and may be in the process of detachment from the basement membrane. We have found endothelial cells with a simlilar morphology in the Tunica vasulosa lentis, a dense capillary plexus associated with the posterior half of the developing lens, after the onset of vascular regression in this system.

The induction of apoptosis by PSI was not restricted to endothelial cells but was observed throughout all the layers of the CAM. In addition to the effects on the vasculature in terms of occlusion and vessel wall destruction, the semi-thin and ultra-thin sections in some cases revealed an increase of the total CAM thickness (Fig. 8 e, g). In sections taken from control CAMs, however, only occasional disruptions of the amniotic epithelium was seen in response to the discs (Fig. 8 f, h). Table II summarizes the results of the CAM experiments in terms of the number of CAMs with non-perfused areas beneath the methylcellulose discs.

**Table II**

| Sample | µg/disc | %CAMs with non-perfused areas |
|---|---|---|
| PSI | 3.1 | 85.7 (12/14) |
| PSI | 1.0 | 56.3 (18/32) |
| PSI | 0.31 | 0(0/10) |
| Leupeptin | 3.1 | 0 (0/26) |
| DMSO | * | 0(0/8) |

| | | |
|---|---|---|
| # CAMs were evalutated microscopically after Luconyl Blue injection to assess the extend of perfusion through the CAM tissue in the area beneath the methycellulose disc. Areas remaining devoid of blue color were scored as positive. The number of CAMs scored as positive in relation to the total number of CAMs examined is given in parenthesis. * The final amount of DMSO pre disc was equal to the volume of DMSO used for the PSI or Leupeptin containing methylcellulose discs. | | |

## Claims

1. Use of a proteasome-inhibitor for the manufacture of an agent for inducing apoptosis in proliferating endothelial or epithelial cells, wherein said proteasome-inhibitor is represented by the general formula (I):
Z-R₁-R₂-R₃-R₄-X
wherein R₁ is selected from leucine, valine and isoleucine, R₂ is selected from glutamine, glutamic acid and blocked derivates thereof, R₃ is selected from alanine and glycine and R₄ is selcected from leucine, valine and isoleucine, Z is an amino group or a protected amino group and X is a carboxy-group, an aldehyde group, a sulfon group or a boronate group.

2. The use of claim 1 wherein R₁ is isoleucine.

3. The use of claim 1 wherein R₂ is a glutamic acid ester, particularly glutamic acid t-butyl-ester.

4. The use of any one of the preceding claims wherein R₃ is alanine.

5. The use of preceding claims wherein R₄ is leucine.

6. The use of preceding claims wherein Z is a carbobenzoxy group.

7. The use of preceding claims wherein the coumpound (I) is carbobenzoxy-L-isoleucyl-*y*-t-butyl-L-glutamyl-L-alanyl-L-leucinal (PSI).

8. The use of any preceding claim wherein the compound has a selectivity for proliferating endothelial or epithelial cells versus quiescent endothelial cells of at least the factor 10.

9. The use of claim 8 wherein the selectivity is at least the factor 100.

10. The use of any one of the preceding claims wherein the apoptosis is incuded by stimulating caspase-3 activity.

11. The use of any one of the preceding claims for the manufacture of an anti-angiogenic agent.

12. The use of claim 11 for the manufacture of an agent against a disorder selected from endothelial tumors, diabetic retinopathy, rheumatoid arthritis, psoriasis and AIDS.

13. A method for inducing apoptosis in proliferating endothelial or epithelial cells comprising administering a subject in need thereof a therapeutically effective amount of a protease-inhibitor having the general formula (I) as defined in claim 1.

14. Use of a proteasome-inhibitor for the manufacture of an agent for inducing apoptosis in leukemic cells having a high expression level of c-myc, wherein said proteasome-inhibitor is represented by the general formula (I) as defined in claim 1.
